# FASCICULE DE BREVET EUROPEEN

(11) **EP 0 966 954 B1**
(45) Date de publication et mention de la délivrance du brevet: **20.02.2002**
(21) Numéro de dépôt: 99401429.8
(22) Date de dépôt: 11.06.1999
(51) Int. Cl.: A61K 7/48, A61K 7/06, A61K 7/42

(54) **Compositions cosmétiques photoprotectrices, contenant un nanopigment d'oxyde métallique et un terpolymère acrylique et utilisation de ces compositions pour protéger les matières kératiniques contre le rayonnement ultraviolet**
Kosmetische Sonnenschutzmittel enthaltend ein Metalloxyd-Nanopigment und ein acrylisches Terpolymer und Verwendung dieser Mittel zum Schutz von Keratinmaterialen gegen UV-Strahlung
Cosmetic sunscreen compositions containing a metal oxide nanopigment and an acrylic terpolymer and use of these compositions for protection keratinic materials against UV-radiation

(30) Priorité: 15.06.1998 FR 9807511
(43) Date de publication de la demande: 29.12.1999
(73) Titulaire: L'OREAL, 75008 Paris (FR)
(72) Inventeur: Candau, Didier, 91570 Bièvres (FR); Hansenne, Isabelle, Westfield, New Jersey 07090 (US)
(74) Mandataire: Casalonga, Axel

(56) Documents cités:
- EP-A- 0 350 414
- EP-A- 0 577 526
- WO-A-93/24544

## Description

La présente invention concerne des compositions cosmétiques photoprotectrices contenant au moins un nanopigment d'oxyde métallique et un terpolymère acrylique ainsi que l'utilisation de ces compositions pour protéger les matières kératiniques, notamment la peau ou les cheveux, contre le rayonnement ultraviolet.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques Il est donc souhaitable de filtrer aussi le rayonnement UV-A.

De nombreux filtres solaires organiques capables d'absorber plus ou moins sélectivement les rayons UV-A et/ou UV-B nocifs ont été proposés à ce jour dans le domaine de la cosmétique.

Cependant, on cherche à diminuer la concentration des filtres solaires organiques de synthèse qui peuvent provoquer sur l'organisme des effets secondaires défavorables, en les remplaçant totalement ou partiellement par des pigments minéraux et notamment par des nanopigments d'oxydes métalliques comme les oxydes de titane, cérium, zirconium, zinc et fer.

L'addition de nanopigments d'oxydes métalliques permet également d'augmenter le pouvoir photoprotecteur des compositions cosmétiques contenant des filtres UV classiques.

De plus en plus, le consommateur apprécie d'utiliser des compositions cosmétiques légères et faciles à appliquer sous une forme liquide épaissie telle qu'un lait, une crème, un gel, un gel-crème. Ce type de présentation correspond à une préoccupation pratique pour l'utilisateur : faciliter la prise du produit hors de son conditionnement sans perte significative, limiter la diffusion du produit à la zone locale d'application et pouvoir l'utiliser dans des quantités suffisantes pour obtenir l'effet recherché.

Néanmoins dans ce type de support, et notamment dans les supports du type gel-crème, qui sont particulièrement agréables d'un point de vue cosmétique car ils ne contiennent pas d'émulsifiant classique, il est difficile d'introduire des nanopigments qui donnent une émulsion stable et homogène. On observe une mauvaise dispersion des nanopigments dans des supports contenant par exemple comme épaississant-gélifiant un polymère comportant dans sa chaîne une partie hydrophile et une partie hydrophobe constituée d'une chaîne grasse, comme le produit "PEMULEN TR1" commercialisé par la société GOODRICH.

La demande de brevet EP-A-0 350 414 décrit l'utilisation de certains terpolymères acryliques comme agents épaississants dans des compositions, notamment cosmétiques, contenant des pigments.

La demanderesse a découvert de manière surprenante, et ceci fait l'objet de la présente invention, une nouvelle famille de polymères épaississants et/ou gélifiants qui permet d'obtenir des compositions cosmétiques stables et homogènes contenant des nanopigments d'oxydes métalliques en améliorant la dispersion de ces derniers dans les supports utilisés.

La présente invention a donc pour objet des compositions cosmétiques protégeant en particulier la peau ou les cheveux contre le rayonnement ultraviolet et contenant dans un support aqueux cosmétiquement acceptable, au moins un nanopigment d'oxyde métallique et un terpolymère acrylique que l'on définira plus en détail dans la suite de la description.

Ce polymère permet notamment de préparer des compositions aqueuses ou hydro-organiques contenant des solvants cosmétiquement acceptables, allant des produits légèrement gélifiés à des produits très consistants.

Les avantages de ce terpolymère sont d'être stable en milieu électrolyte, d'avoir un très bon pouvoir épaississant à pH égal ou supérieur à 5,5 permettant d'atteindre un bon niveau de viscosité et de pouvoir utiliser des concentrations élevées en alcool.

Ce polymère permet de réaliser des gels homogènes, ayant une bonne facilité d'étalement, doux et glissants à l'application et stables à la conservation.

Le terpolymère acrylique utilisé conformément à l'invention est soluble ou gonflable dans les alcalis. Il est caractérisé par le fait qu'il comprend :
a) 20 à 70% en poids, de préférence 25 à 55% en poids, d'un acide carboxylique à insaturation α, β-monoéthylénique ;
b) 20 à 80% en poids, de préférence 30 à 65% en poids, d'un monomère à insaturation monoéthylénique non tensio-actif différent de a) et
c) 0,5 à 60% en poids, de préférence 10 à 50% en poids, d'un monomère uréthane non-ionique qui est le produit de réaction d'un tensio-actif non-ionique monohydrique avec un monoisocyanate à insaturation monoéthylénique.

L'acide carboxylique à insaturation α, β-monoéthylénique a) peut être choisi parmi de nombreux acides et en particulier l'acide acrylique, l'acide méthacrylique, l'acide itaconique et l'acide maléique. L'acide méthacrylique est préféré. Une large proportion d'acide est essentielle pour donner une structure polymère qui se solubilise et donne un épaississant par réaction avec un composé alcalin comme l'hydroxyde de sodium, les alcanolamines, l'amino méthyl propanol, ou l'amino méthyl propanediol.

Le terpolymère doit aussi contenir une proportion importante indiquée ci-dessus d'un monomère b) à insaturation monoéthylénique qui n'a pas de propriété tensio-active. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensio-actifs non-ioniques monohydriques utilisés pour obtenir le monomère uréthane non-ionique c) sont bien connus et sont généralement des composés hydrophobes alcoxylés contenant un oxyde d'alkylène formant la partie hydrophile de la molécule. Les hydrophobes sont généralement constitués par un alcool aliphatique ou un alkylphénol dans lesquels une chaîne carbonée contenant au moins six atomes de carbone constitue la partie hydrophobe du tensio-actif.

Les tensio-actifs non-ioniques monohydriques préférés ont pour formule : 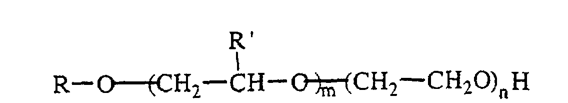 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n+m=5-150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R' préféré est le groupe méthyle.

Le monoisocyanate à insaturation monoéthylénique utilisé pour former le monomère uréthane non-ionique c) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Le monoisocyanate monoéthylénique préféré est l'α,α-diméthyl-m-isopropényl-benzylisocyanate.

Le terpolymère acrylique défini ci-dessus est obtenu par copolymérisation en émulsion aqueuse des composants a), b) et c) qui est tout à fait usuelle et décrite dans la demande de brevet EP-A-0 173 109.

A titre de terpolymères pouvant être utilisés selon l'invention, on peut citer le produit de réaction de l'acide méthacrylique comme composant a), de l'acrylate d'éthyle comme composant b) et d'un macromonomère uréthane non-ionique comme composant c), ayant la structure suivante : 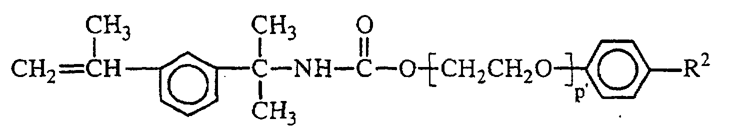 dans laquelle p' va de 6 à 150 et est égal de préférence à 30 et R² est un radical alkyle en C₈-C₁₃ tel que décrit dans l'exemple 3 de la demande de brevet EP-A-0 173 109.

Le terpolymère acrylique préféré utilisé selon l'invention est obtenu à partir d'acide méthacrylique comme composant a), d'acrylate de méthyle comme composant b) et d'un macromonomère uréthane non-ionique comme composant (c), ayant la structure suivante : 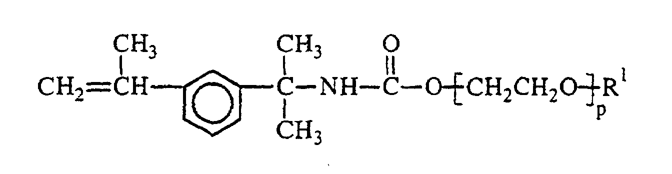 dans laquelle p va de 6 à 150 et R¹ est un radical alkyle en C₁₈-C₂₆, de préférence en C₂₀-C₂₄ linéaire, d'origine végétale, tel que le radical docosyle.

Le terpolymère acrylique est présent dans les compositions cosmétiques photoprotectrices de l'invention dans des concentrations allant de 0,01 à 10% en poids par rapport au poids total de la composition et préférentiellement de 0,1 à 5% en poids.

Les compositions conformes à l'invention contiennent en outre au moins un nanopigment d'oxyde métallique choisi parmi les oxydes de titane, cérium, zirconium, zinc, fer ou leurs mélanges.

On entend par nanopigment un pigment dont la taille moyenne des particules élémentaires est supérieure à 5 nm et inférieure à 100 nm. Selon un mode préféré de réalisation de l'invention, cette taille est inférieure à 50 nm.

Les nanopigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme de "silicones" englobe également les silanes nécessaires à leur préparation, en particulier, les alkyl silanes.

Les silicones utilisées pour l'enrobage des nanopigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydrogénosiloxanes.

Bien entendu, les nanopigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et"MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et" UVT 14/4" de la société KEMIRA,
- d'alumine et de stéarate d'aluminium tels que le produit "MICROTITANIUM DIOXIDE MT 100 T, MT 100 TX, MT 100 Z" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR 351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS "de la société TAYCA,
- de silice, d'alumine, de stéarate d'aluminium et traités par une silicone tels que le produit "STT-30-DS" de la société TITAN KOGYO,
- d'alumine et traités par une silicone tels que les produits "TIPAQUE TTO-55 (S)" de la société ISHIHARA, ou "UV TITAN M 262" de la société KEMIRA,
- de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres nanopigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO₂ traité par un polydiméthylsiloxane et dont la taille moyenne des particules élémentaires est de 21 nm tel que celui vendu sous la dénomination commerciale "70250 Cardre UF TiO₂SI3" par la société CARDRE, le TiO₂ anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les oxydes de titane non enrobés sont par exemple vendus par la société TAYCA sous les dénominations commerciales "MICROTITANIUM DIOXIDE MT 500 B" ou "MICROTITANIUM DIOXIDE MT 600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les oxydes de zinc non enrobés sont par exemple vendus par la société SUMITOMO sous la dénomination "ULTRA FINE ZINC OXIDE POWDER", par la société PRESPERSE sous la dénomination "FINEX 25" ou par la société IKEDA sous la dénomination "MZO-25", ou par la société SUNSMART sous la dénomination "Z-COTE".

Les oxydes de zinc enrobés sont par exemple ceux vendus sous la dénomination "Z-COTE HP1" par la société SUNSMART.

L'oxyde de cérium non enrobé est vendu sous la dénomination "COLLOIDAL CERIUM OXIDE" par la société RHONE POULENC.

Les oxydes de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les oxydes de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

Selon l'invention, les nanopigments d'oxyde de titane, enrobés ou non enrobés, sont particulièrement préférés.

L'oxyde de titane peut se présenter sous forme rutile, anatase ou amorphe, mais de préférence sous forme rutile et/ou anatase.

Les nanopigments peuvent être présents dans les compositions conformes à l'invention dans des concentrations qui sont généralement comprises entre 0,1 % et 30 % en poids, et de préférence comprises entre 0,5% et 10 % en poids, par rapport au poids total de la composition.

Outre les nanopigments, les compositions selon l'invention peuvent aussi contenir des pigments classiques non colorés de granulométrie usuelle comprise entre 100 et 700 nm tels que l'oxyde de zinc le dioxyde de titane "F.F. HOMBITAN" de taille moyenne de particules élémentaires 400 nm vendu par la société SACHTLEBEN CHEMIE GmbH, l'oxyde de zinc "NEIGE" vendu par la société LAMBERT RIVIERE, ou encore des pigments colorés comme les oxydes de fer "FDC RED 40 (37011/90119)" vendu par la société ANSTEAD, "SICOMET BRUN ZP 3569" et "SICOVIT JAUNE 10 E 172" vendus par la société BASF.

Les compositions cosmétiques selon l'invention peuvent bien entendu contenir un ou plusieurs filtres solaires organiques absorbant dans l'UV-A et/ou l'UV-B, hydrophiles ou lipophiles. Ces filtres peuvent être notamment choisis parmi les cinnamates, les salicylates, les dérivé du benzylidène camphre, les dérivés de triazine, les dérivés de la benzophénone, les dérivés de benzotriazole, les dérivés de β,β-diphénylacrylate, les dérivés du dibenzoylméthane, les p-aminobenzoates, les polymères filtres et silicones filtres décrits dans la demande WO-93/04665. D'autres exemples de filtres organiques sont donnés dans la demande de brevet BP-A 0 487 404. Ces filtres organiques peuvent être présents dans les compositions selon l'invention à des concentrations comprises entre 0,1 et 30% en poids, par rapport au poids total de la composition.

Les compositions selon l'invention peuvent également contenir des agents de bronzage et/ou de brunissage artificiels de la peau (agents autobronzants), tels que par exemple de la dihydroxyacétone (DHA).

Les compositions de l'invention contiennent un milieu aqueux cosmétiquement acceptable. Elles présentent un pH pouvant aller de 3,5 à 11, de préférence compris entre 5,5 et 11 et encore plus préférentiellement de 5,5 à 8,5.

Le milieu cosmétiquement acceptable des compositions selon l'invention est plus particulièrement constitué d'eau et éventuellement de solvants organiques cosmétiquement acceptables.

Les solvants organiques peuvent représenter de 0,5 à 90% du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple les mono-alcools inférieurs linéaires ou ramifiés ayant de 1 à 8 atomes de carbone, les polyéthylène glycols ayant de 6 à 80 unités d'oxyde d'éthylène et les polyols.

Comme solvants organiques amphiphiles, on peut citer les dérivés de polypropylène glycol (PPG) comme les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle, le malate de dioctyle.

Afin que les compositions cosmétiques de l'invention soient plus agréables à utiliser (plus douces à l'application, plus nourrissantes, plus émollientes), il est possible d'ajouter une phase grasse dans le milieu de ces compositions.

La phase grasse peut représenter jusqu'à 50% du poids total de la composition.

Cette phase grasse peut comporter une huile ou une cire ou leurs mélanges, et peut comprendre également des acides gras, des alcools gras et des esters d'acides gras. Les huiles peuvent être choisies parmi les huiles animales, végétales, minérales ou de synthèse et notamment parmi l'huile de vaseline, l'huile de paraffine, les isoparaffines, les poly-α-oléfines, les huiles fluorées et perfluorées. De même, les cires peuvent être choisies parmi les cires animales, fossiles, végétales, minérales ou de synthèse connues en soi.

Les compositions de l'invention peuvent contenir des adjuvants habituels dans le domaine cosmétique comme d'autres gélifiants et/ou épaississants classiques ; d'autres polymères anioniques ; des tensio-actifs ; des agents hydratants ; des émollients ; des actifs hydrophiles ou lipophiles comme des céramides ; des agents anti-radicaux libres ; des séquestrants ; des antioxydants ; des conservateurs ; des agents alcalinisants ou acidifiants ; des parfums ; des charges ; des matières colorantes, des silicones volatiles ou non, modifiées ou non, des réducteurs. Les quantités de ces différents adjuvants sont celles classiquement utilisées dans les domaines considérés.

Bien entendu, le spécialiste veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, en particulier sous forme de lotions, de dispersions vésiculaires, d'émulsions simples ou complexes (H/E, E/H, H/E/H, E/H/E) telles que des crèmes ou des laits, de gels aqueux ou hydroalcooliques ou de gels-crèmes, sous forme de poudres, de pâtes, et éventuellement être conditionnées en aérosol et se présenter sous forme de mousses ou de sprays. De préférence, ces compositions se présentent sous la forme d'un gel-crème. Elles sont préparées selon les méthodes usuelles.

Les compositions cosmétiques de l"invention peuvent être utilisées comme compositions protectrices de l'épiderme humain ou des cheveux contre les rayons ultraviolets, comme compositions antisolaires ou comme produits de maquillage.

Lorsque la composition cosmétique selon l"invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous formé d'émulsion, telle qu'une crème ou un lait, sous forme de pommade, de gel, de gel-crème, de mousse aérosol ou de spray.

Lorsque la composition cosmétique selon l'invention est utilisée pour la protection des cheveux, elle peut se présenter sous forme de lotion, de gel, d'émulsion, de dispersion vésiculaire non ionique et constituer par exemple un shampooing, une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile-dans-eau ou eau-dans-huile, des dispersions vésiculaires non ioniques ou encore des suspensions.

Un autre objet de la présente invention réside dans un procédé de traitement non-thérapeutique cosmétique de la peau, des cheveux, des cils, des sourcils ou des muqueuses destiné à les protéger contre les effets des rayons UV, consistant à appliquer sur ceux-ci une quantité efficace d'une composition cosmétique telle que définie ci-dessus.

Des exemples concrets, mais nullement limitatifs, illustrant l'invention, vont maintenant être donnés.

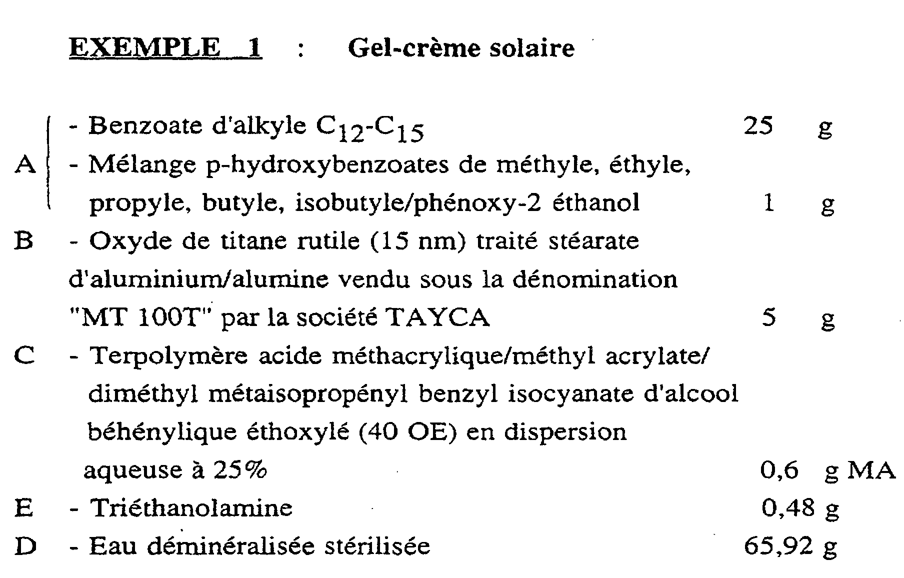

Toutes les étapes de préparation de ce gel-crème sont réalisées à froid.
1 - On disperse B dans A à l'aide d'un Ultra Turrax T25 à 10 000 tr/min
2 - On pèse C + D dans un bécher
3 - On verse la préparation (B + A) sur (C + D) en agitant avec l'Ultra Turrax T25 à 10 000 tr/min
4 - On verse E et on homogénéise.

On obtient un gel-crème homogène et stable s'étalant bien sur la peau.

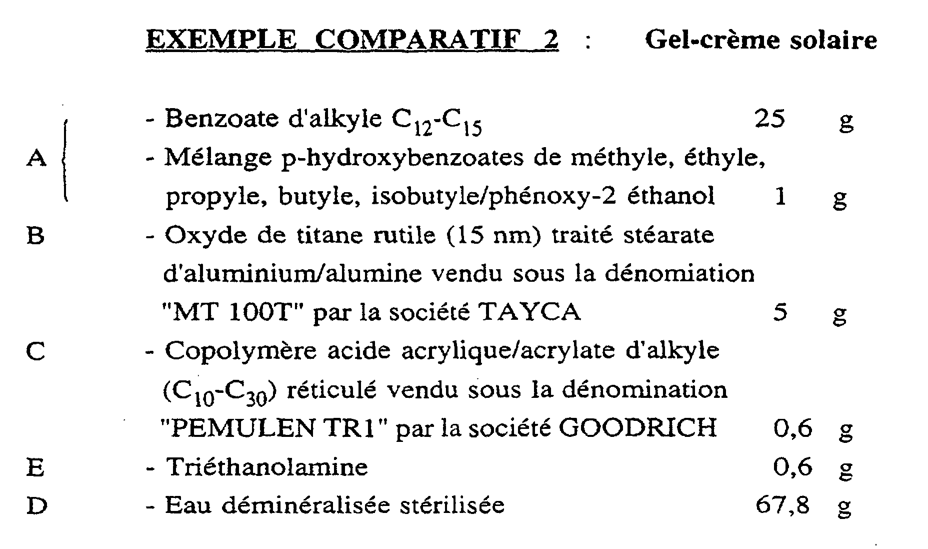

Toutes les étapes de préparation de ce gel-crème sont réalisées à froid.
1 - On disperse B dans A à l'aide d'un Ultra Turrax T25 à 10 000 tr/min
2 - On verse D sur C en homogénéisant avec un Moritz Turbo Lab 2100, 500 tr/min jusqu'à dissolution de C
3 - On verse la préparation (B + A) sur (C + D) en agitant avec l'Ultra Turrax T25 à 10 000 tr/min
4 - On verse E et on homogénéise.

On obtient un gel-crème non homogène, instable comportant de gros globules huileux et de gros amas de pigment.

### EXEMPLE 3 : Gel-crème solaire haute protection

- 4 tert.butyl 4'-méthoxy dibenzoylméthane ("PARSOL 1789" vendu par la société ROCHE) 2 g
- Acide benzène 1,4-di(3-méthylidène-10-campho-sulfonique) en solution aqueuse à 33% 1,5 g
- 2-cyano-3,3-diphénylacrylate de 2-éthylhexyle ("UVINUL N 539" vendu par la société BASF) 10 g
- Terpolymère acide méthacrylique/méthyl acrylate/diméthyl métaisopropényl benzyl isocyanate d'alcool béhénylique éthoxylé (40 OE) en dispersion aqueuse à 25% 3 g MA
- Dioxyde de titane traité par l'octyl triméthyl silane vendu sous la dénomination "T805" par la société DEGUSSA SILICES 5 g
- Isohexadécane 10 g
- Stéarate d'oligomère d'acide 12-hydroxystéarique 0,5 g
- Hydratants 8 g
- Séquestrant q.s.
- Triéthanolamine q.s. pH 7
- Eau déminéralisée stérilisée qsp 100 g

On obtient un gel-crème stable, homogène et s'étalant bien sur la peau.

## Revendications

1. Composition cosmétique pour la photoprotection des matières kératiniques, **caractérisée par le fait qu'**elle comprend dans un support aqueux cosmétiquement acceptable, au moins un nanopigment d'oxyde métallique et un terpolymère acrylique comprenant :
a) 20 à 70% en poids, et de préférence 25 à 55% en poids, d'un acide carboxylique à insaturation α, β-monoéthylénique ;
b) 20 à 80% en poids, et de préférence 30 à 65% en poids, d'un monomère à insaturation monoéthylénique non tensio-actif différent de a) et
c) 0,5 à 60% en poids, et de préférence 10 à 50% en poids, d'un monomère uréthane non-ionique qui est le produit de réaction d'un tensio-actif non-ionique monohydrique avec un monoisocyanate à insaturation monoéthylénique.

2. Composition selon la revendication 1, **caractérisée par le fait que** l'acide carboxylique à insaturation α, β-monoéthylénique a) est choisi parmi l'acide acrylique, l'acide méthacrylique, l'acide itaconique et l'acide maléique.

3. Composition selon la revendication 2, **caractérisée par le fait que** l'acide carboxylique à insaturation α, β-monoéthylénique a) est l'acide méthacrylique.

4. Composition selon l'une quelconque des revendications 1 à 3, **caractérisée par le fait que** le monomère à insaturation monoéthylénique non tensio-actif b) est choisi parmi les acrylates et méthacrylates d'alkyle en C₁-C₄, le styrène, le vinyltoluène, l'acétate de vinyle, l'acrylonitrile et le chlorure de vinylidène.

5. Composition selon la revendication 4, **caractérisée par le fait que** le monomère à insaturation monoéthylénique non tensio-actif est l'acrylate de méthyle ou d'éthyle.

6. Composition selon l'une quelconque des revendications 1 à 5, **caractérisée par le fait que** le tensio-actif non-ionique monohydrique utilisé pour obtenir le monomère uréthane non-ionique c) a pour formule : 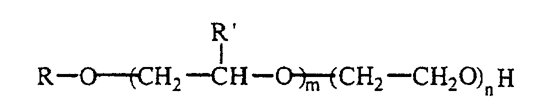 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

7. Composition selon la revendication 6, **caractérisée par le fait que** R est choisi parmi les radicaux dodécyle, alkyle en C₁₈-C₂₆ et alkyl (C₈-C₁₃) phényle, m = 0 et n est un nombre moyen allant de 5 à 150.

8. Composition selon l'une quelconque des revendications 1 à 7, **caractérisée par le fait que** le monoisocyanate à insaturation monoéthylénique utilisé pour former le monomère uréthane non-ionique c) est l'α,α-diméthyl m-isopropényl benzyl isocyanate.

9. Composition selon l'une quelconque des revendications 1 à 8, **caractérisée par le fait que** le terpolymère acrylique est obtenu en dispersion aqueuse à partir d'acide méthacrylique comme composant a), d'acrylate de méthyle comme composant b) et d'un macromonomère uréthane non-ionique de structure suivante : 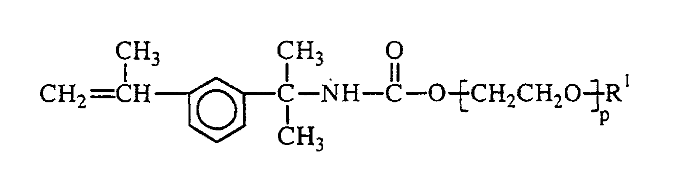 dans laquelle p va de 6 à 150 et R¹ est un radical alkyle en C₁₈-C₂₆, de préférence en C₂₀-C₂₄ linéaire, d'origine végétale, tel que le radical docosyle.

10. Composition selon l'une quelconque des revendications 1 à 9, **caractérisée par le fait que** le terpolymère acrylique est présent dans des concentrations allant de 0,01 à 10% en poids, et de préférence de 0,1 à 5% en poids, par rapport au poids total de la composition.

11. Composition selon l'une quelconque des revendications 1 à 10, **caractérisée par le fait que** les nanopigments d'oxydes métalliques ont une taille moyenne de particules élémentaires supérieure à 5 nm et inférieure à 100 nm.

12. Composition selon la revendication 11, **caractérisée par le fait que** les nanopigments d'oxydes métalliques ont une taille moyenne de particules élémentaires inférieure à 50 nm.

13. Composition selon l'une quelconque des revendications 1 à 12, **caractérisée par le fait que** les oxydes métalliques sont choisis parmi les oxydes de titane, cérium, zirconium, zinc, fer et leurs mélanges.

14. Composition selon la revendication 1 à 13, **caractérisée par le fait que** l'oxyde métallique est le dioxyde de titane.

15. Composition selon l'une quelconque des revendications 1 à 14, **caractérisée par le fait que** les nanopigments d'oxydes métalliques sont des pigments enrobés ayant subi un ou plusieurs traitements de surface de nature chimique, électronique, mécanochimique ou mécanique avec des composés choisis parmi les aminoacides, la cire d'abeilles, les acides gras, les alcools gras, les tensio-actifs anioniques, les lécithines, les sels de sodium, potassium, zinc, fer ou aluminium d'acides gras, l'hexamétaphosphate de sodium, les alcoxydes métalliques, les polyéthylènes, les silicones, les protéines, les alcanolamines, les oxydes de silicium et les oxydes métalliques.

16. Composition selon la revendication 15, **caractérisée par le fait que** les nanopigments d'oxydes métalliques enrobés sont des pigments de dioxyde de titane enrobés de silice, de silice et d'alumine, de silice et d'oxyde de fer, d'alumine, d'alumine et de stéarate d'aluminium, d'alumine et de laurate d'aluminium, d'oxyde de fer et de stéarate de fer, d'oxyde de zinc et de stéarate de zinc, de triéthanolamine, d'acide stéarique, d'hexamétaphosphate de sodium, d'octyl triméthyl silane, de polydiméthylsiloxane, de polyméthylhydrogénosiloxane, d'alumine et de silicone, de silice et d'alumine et de silicone, ou de silice et d'alumine et de stéarate d'aluminium et de silicone.

17. Composition selon l'une quelconque des revendications 1 à 16, **caractérisée par le fait que** le ou les nanopigments d'oxydes métalliques sont présents dans des concentrations allant de 0,1 à 30% en poids, et de préférence de 0,5 à 10% en poids, par rapport au poids total de la composition.

18. Composition selon l'une quelconque des revendications 1 à 17, **caractérisée par le fait qu'**elle présente un pH allant de 3,5 à 11, de préférence de 5,5 à 11 et encore plus préférentiellement de 5,5 à 8,5.

19. Composition selon l'une quelconque des revendications 1 à 18, **caractérisée par le fait qu'**elle se présente sous forme d'émulsion, de lotion, de gel, de dispersion vésiculaire, de pâte, de poudre ou est conditionnée en aérosol et se présente sous forme de mousse ou de spray.

20. Composition selon la revendication 19, **caractérisée par le fait qu'**elle se présente sous forme d'émulsion et constitue un gel-crème.

21. Composition selon l'une quelconque des revendications 1 à 20, **caractérisée par le fait que** le milieu cosmétiquement acceptable est constitué d'eau ou d'eau et au moins un solvant organique choisi dans le groupe constitué par les solvants organiques hydrophiles, lipophiles, amphiphiles ou leurs mélanges.

22. Composition selon l'une quelconque des revendications 1 à 21, **caractérisée par le fait qu'**elle comprend en outre au moins un adjuvant cosmétique usuel choisi parmi les corps gras, les gélifiants et/ou épaississants classiques, les polymères anioniques autres que le terpolymère acrylique défini dans la revendication 1, les tensio-actifs, les agents hydratants, les émollients, les filtres solaires, les pigments d'oxydes métalliques usuels, les actifs hydrophiles ou lipophiles comme les céramides ou la dihydroxyacétone, les agents anti-radicaux libres, les séquestrants, les antioxydants, les conservateurs, les agents alcalinisants ou acidifiants, les parfums, les charges, les matières colorantes, les silicones et les réducteurs.

23. Procédé de traitement non-thérapeutique cosmétique pour la protection de la peau, des cheveux, des cils, des sourcils ou des muqueuses, **caractérisé par le fait qu'**on applique sur ces derniers une quantité efficace d'une composition telle que définie selon l'une quelconque des revendications 1 à 22.

## Patentansprüche

1. Kosmetische Zusammensetzung zum Lichtschutz von Keratinsubstanzen, **dadurch gekennzeichnet, daß** sie in einem kosmetisch akzeptablen, wäßrigen Träger mindestens ein Nanopigment eines Metalloxids und ein Acrylterpolymer enthält, welches aufweist:
a) 20 bis 70 Gew.-% und vorzugsweise 25 bis 55 Gew.-% einer Carbonsäure mit α,β-monoethylenischer Doppelbindung;
b) 20 bis 80 Gew.-% und vorzugsweise 30 bis 65 Gew.-% eines nicht grenzflächenaktiven Monomers mit monoethylenischer Doppelbindung, das von a) verschieden ist, und
c) 0,5 bis 60 Gew.-% und vorzugsweise 10 bis 50 Gew.-% eines nichtionischen Urethanmonomers, das bei der Umsetzung eines nichtionischen grenzflächenaktiven Stoffes mit einer Hydroxygruppe mit einem Monoisocyanat mit monoethylenischer Doppelbindung entsteht.

2. Zusammensetzung nach Anspruch 1, **dadurch gekennzeichnet, daß** die Carbonsäure mit α,β-monoethylenischer Doppelbindung a) unter Acrylsäure, Methacrylsäure, Itaconsäure und Maleinsäure ausgewählt ist.

3. Zusammensetzung nach Anspruch 2, **dadurch gekennzeichnet, daß** die α,β-monoethylenisch ungesättigte Carbonsäure a) die Methacrylsäure ist.

4. Zusammensetzung nach einem der Ansprüche 1 bis 3, **dadurch gekennzeichnet, daß** das Monomer mit monoethylenischer Doppelbindung b), das kein grenzflächenaktiver Stoff ist, unter den C₁₋₄-Alkylacrylaten und C₁₋₄-Alkylmethacrylaten, Styrol, Vinyltoluol, Vinylacetat, Acrylnitril und Vinylidenchlorid ausgewählt ist.

5. Zusammensetzung nach Anspruch 4, **dadurch gekennzeichnet, daß** das nicht grenzflächenaktive monoethylenisch ungesättigte Monomer das Methylacrylat oder das Ethylacrylat ist.

6. Zusammensetzung nach einem der Ansprüche 1 bis 5, **dadurch gekennzeichnet, daß** der zur Herstellung des nichtionischen Urethanmonomers c) verwendete nichtionische grenzflächenaktive Stoff mit einer Hydroxygruppe die folgende Formel aufweist: 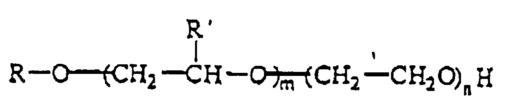 wobei R ein C₆₋₃₀-Alkylgruppe oder eine C₈₋₃₀-Aralkylgruppe, R' eine C₁₋₄-Alkylgruppe, n einen Mittelwert von 5 bis 150 und m einen Mittelwert von 0 bis 50 bedeutet, mit der Maßgabe, daß n mindestens so groß wie m ist und n + m = 5 - 150.

7. Zusammensetzung nach Anspruch 6, **dadurch gekennzeichnet, daß** R unter Dodecyl, C₁₈₋₂₆-Alkyl und C₈₋₁₃-Alkylphenyl ausgewählt ist, m = 0 und n einen Mittelwert von 5 bis 150 bedeutet.

8. Zusammensetzung nach einem der Ansprüche 1 bis 7, **dadurch gekennzeichnet, daß** das Monoisocyanat mit monoethylenischer Doppelbindung, das zur Bildung des nichtionischen Urethanmonomers c) verwendet wird, das α,α-Dimethyl-m-isopropenylbenzylisocyanat ist.

9. Zusammensetzung nach einem der Ansprüche 1 bis 8, **dadurch gekennzeichnet, daß** das Acrylterpolymer in wäßriger Dispersion ausgehend von Methacrylsäure als Komponente a), Methylacrylat als Komponente b) und einem nichtionischen Urethanmakromer der folgenden Struktur als Komponente c) hergestellt wird: 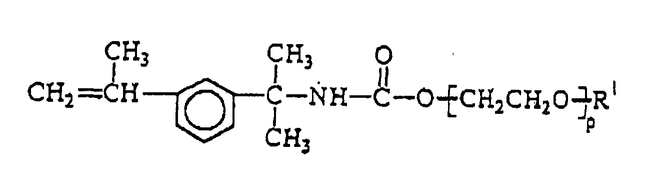 wobei p im Bereich von 6 bis 150 liegt und R¹ eine Alkylgruppe mit 18 bis 26 Kohlenstoffatomen und vorzugsweise eine geradkettige Alkylgruppe mit 20 bis 24 Kohlenstoffatomen pflanzlicher Herkunft ist, wie Docosyl.

10. Zusammensetzung nach einem der Ansprüche 1 bis 9, **dadurch gekennzeichnet, daß** das Acrylterpolymer in Konzentrationen von 0,01 bis 10 Gew.-% und vorzugsweise 0,1 bis 5 Gew.-%, bezogen auf das Gesamtgewicht der Zusammensetzung, vorliegt.

11. Zusammensetzung nach einem der Ansprüche 1 bis 10, **dadurch gekennzeichnet, daß** die Nanopigmente von Metalloxiden eine mittlere Größe der Elementarpartikel über 5 nm und unter 100 nm aufweisen.

12. Zusammensetzung nach Anspruch 11, **dadurch gekennzeichnet, daß** die Nanopigmente von Metalloxiden eine mittlere Größe der Elementarpartikel unter 50 nm aufweisen.

13. Zusammensetzung nach einem der Ansprüche 1 bis 12, **dadurch gekennzeichnet, daß** die Metalloxide unter den Oxiden von Titan, Cer, Zirconium, Zink, Eisen und deren Gemischen ausgewählt sind.

14. Zusammensetzung nach Anspruch 1 bis 13, **dadurch gekennzeichnet, daß** das Metalloxid das Titandioxid ist.

15. Zusammensetzung nach einem der Ansprüche 1 bis 14, **dadurch gekennzeichnet, daß** die Nanopigmente von Metalloxiden umhüllte Pigmente sind, die einer oder mehreren Oberflächenbehandlungen chemischer, elektronischer, mechanischchemischer oder mechanischer Art mit Verbindungen unterzogen wurden, die unter den Aminosäuren, Bienenwachs, Fettsäuren, Fettalkoholen, anionischen grenzflächenaktiven Stoffen, Lecithinen, Natrium-, Kalium-, Zink-, Eisen- oder Aluminiumsalzen von Fettsäuren, Natriumhexametaphosphat, Metallalkoxiden, Polyethylenen, Siliconen, Proteinen, Alkanolaminen, Siliciumoxiden und Metalloxiden ausgewählt sind.

16. Zusammensetzung nach Anspruch 15, **dadurch gekennzeichnet, daß** die umhüllten Nanopigmente von Metalloxiden Pigmente von Titandioxid sind, die mit Siliciumoxid, Siliciumoxid und Aluminiumoxid, Siliciumoxid und Eisenoxid, Aluminiumoxid, Aluminiumoxid und Aluminiumstearat, Aluminiumoxid und Aluminiumlaurat, Eisenoxid und Eisenstearat, Zinkoxid und Zinkstearat, Triethanolamin, Stearinsäure, Natriumhexametaphosphat, Octyltrimethylsilan, Polydimethylsiloxan, Polymethylhydrogensiloxan, Aluminiumoxid und Silicon, Siliciumoxid und Aluminiumoxid und Silicon oder Siliciumoxid und Aluminiumoxid und Aluminiumstearat und Silicon umhüllt sind.

17. Zusammensetzung nach einem der Ansprüche 1 bis 16, **dadurch gekennzeichnet, daß** das Nanopigment oder die Nanopigmente von Metalloxiden in Konzentrationen von 0,1 bis 30 Gew.-% und vorzugsweise 0,5 bis 10 Gew.-%, bezogen auf das Gesamtgewicht der Zusammensetzung, vorliegen.

18. Zusammensetzung nach einem der Ansprüche 1 bis 17, **dadurch gekennzeichnet, daß** sie einen pH-Wert von 3,5 bis 11, vorzugsweise 5,5 bis 11 und noch bevorzugter 5,5 bis 8,5 aufweist.

19. Zusammensetzung nach einem der Ansprüche 1 bis 18, **dadurch gekennzeichnet, daß** sie als Emulsion, Lotion, Gel, Vesikeldispersion, Paste oder Pulver vorliegt oder als Aerosol konfektioniert ist und in Form von Schaum oder Spray vorliegt.

20. Zusammensetzung nach Anspruch 19, **dadurch gekennzeichnet, daß** sie in Form einer Emulsion vorliegt und eine Gelcreme ist.

21. Zusammensetzung nach einem der Ansprüche 1 bis 20, **dadurch gekennzeichnet, daß** das kosmetisch akzeptable Medium aus Wasser oder Wasser und mindestens einem organischen Lösungsmittel besteht, das unter den hydrophilen organischen Lösungsmitteln, lipophilen organischen Lösungsmitteln, amphiphilen organischen Lösungsmitteln oder deren Gemischen ausgewählt ist.

22. Zusammensetzung nach einem der Ansprüche 1 bis 21, **dadurch gekennzeichnet, daß** sie ferner mindestens einen üblichen kosmetischen Zusatzstoff enthält, der unter den Fettsubstanzen, herkömmlichen Gelbildnern und/oder Verdickungsmitteln, anionischen Polymeren, die von dem in Anspruch 1 definierten Acrylterpolymer verschieden sind, grenzflächenaktiven Stoffen, Hydratisierungsmitteln, Emollientien, Sonnenschutzfiltern, herkömmlichen Pigmenten von Metalloxiden, hydrophilen oder lipophilen Wirkstoffen, wie Ceramiden oder Dihydroxyaceton, Mitteln gegen freie Radikale, Maskierungsmitteln, Antioxidantien, Konservierungsmitteln, Alkalisierungsmitteln, Ansäuerungsmitteln, Parfums, Füllstoffen, Farbmitteln, Siliconen und Reduktionsmitteln ausgewählt sind.

23. Verfahren zur nicht therapeutischen kosmetischen Behandlung zum Schutz der Haut, der Haare, der Wimpern, der Augenbrauen oder der Schleimhäute, **dadurch gekennzeichnet, daß** auf diese eine Zusammensetzung nach einem der Ansprüche 1 bis 22 in einer wirksamen Menge aufgebracht wird.

## Claims

1. Cosmetic composition for the photoprotection of keratinous material, **characterized in that** it comprises, in a cosmetically acceptable aqueous support, at least one metal oxide nanopigment and an acrylic terpolymer comprising:
a) 20 to 70% by weight, preferably 25 to 55% by weight, of a carboxylic acid containing α, β-monoethylenic unsaturation;
b) 20 to 80% by weight, preferably 30 to 65% by weight, of a non-surfactant monomer containing monoethylenic unsaturation, which is different from a), and
c) 0.5 to 60% by weight, preferably 10 to 50% by weight, of a nonionic urethane monomer which is the product of reaction of a monohydric nonionic surfactant with a monoisocyanate containing monoethylenic unsaturation.

2. Composition according to Claim 1, **characterized in that** the carboxylic acid containing α,β-monoethylenic unsaturation a) is chosen from acrylic acid, methacrylic acid, itaconic acid and maleic acid.

3. Composition according to Claim 2, **characterized in that** the carboxylic acid containing α,β-mono-ethylenic unsaturation a) is methacrylic acid.

4. Composition according to any one of Claims 1 to 3, **characterized in that** the non-surfactant monomer containing monoethylenic unsaturation b) is chosen from (C₁-C₄)alkyl acrylates and methacrylates, styrene, vinyltoluene, vinyl acetate, acrylonitrile and vinylidene chloride.

5. Composition according to Claim 4, **characterized in that** the non-surfactant monomer containing monoethylenic unsaturation is methyl or ethyl acrylate.

6. Composition according to any one of Claims 1 to 5, **characterized in that** the monohydric nonionic surfactant used to obtain the nonionic urethane monomer c) has the formula: 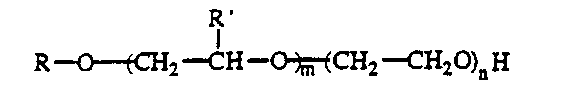 in which R is a C₆-C₃₀ alkyl or C₈-C₃₀ aralkyl group, R' is a C₁-C₄ alkyl group, n is an average number ranging from 5 to 150 and m is an average number ranging from 0 to 50, with the condition that n is at least as large as m and that n + m = 5-150.

7. Composition according to Claim 6, **characterized in that** R is chosen from dodecyl, C₁₈-C₂₆ alkyl and (C₈-C₁₃)alkylphenyl radicals, m = 0 and n is an average number ranging from 5 to 150.

8. Composition according to any one of Claims 1 to 7, **characterized in that** the monoisocyanate containing monoethylenic unsaturation used to form the nonionic urethane monomer c) is α,α-dimethyl-m-isopropenyl benzyl isocyanate.

9. Composition according to any one of Claims 1 to 8, **characterized in that** the acrylic terpolymer is obtained as an aqueous dispersion from methacrylic acid as component a), from methyl acrylate as component b) and from a nonionic urethane macromonomer of the following structure: 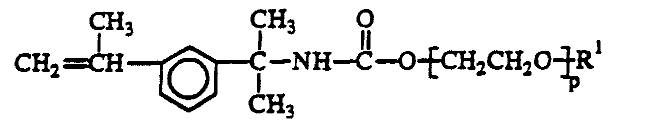 in which p ranges from 6 to 150 and R¹ is a linear C₁₈-C₂₆, preferably C₂₀-C₂₄, alkyl radical of plant origin, such as the docosyl radical.

10. Composition according to any one of Claims 1 to 9, **characterized in that** the acrylic terpolymer is present in concentrations ranging from 0.01 to 10% by weight, and preferably from 0.1 to 5% by weight, relative to the total weight of the composition.

11. Composition according to any one of Claims 1 to 10, **characterized in that** the metal oxide nanopigments have a mean elementary particle size of greater than 5 nm and less than 100 nm.

12. Composition according to Claim 11, **characterized in that** the metal oxide nanopigments have a mean elementary particle size of less than 50 nm.

13. Composition according to any one of Claims 1 to 12, **characterized in that** the metal oxides are chosen from titanium, cerium, zirconium, zinc and iron oxides, and mixtures thereof.

14. Composition according to Claims 1 to 13,
**characterized in that** the metal oxide is titanium dioxide.

15. Composition according to any one of Claims 1 to 14, **characterized in that** the metal oxide nanopigments are coated pigments which have undergone one or more surface treatments of chemical, electronic, mechano-chemical or mechanical nature with compounds chosen from amino acids, beeswax, fatty acids, fatty alcohols, anionic surfactants, lecithins, sodium, potassium, zinc, iron or aluminium salts of fatty acids, sodium hexametaphosphate, metal alkoxides, polyethylenes, silicones, proteins, alkanolamines, silicon oxides and metal oxides.

16. Composition according to Claim 15, **characterized in that** the coated metal oxide nanopigments are titanium dioxide pigments coated with silica, with silica and alumina, with silica and iron oxide, with alumina, with alumina and aluminium stearate, with alumina and aluminium laurate, with iron oxide and iron stearate, with zinc oxide and zinc stearate, with triethanolamine, with stearic acid, with sodium hexametaphosphate, with octyltrimethylsilane, with polydimethylsiloxane, with polymethylhydrogenosiloxane, with alumina and silicone, with silica and alumina and silicone, or with silica and alumina and aluminium stearate and silicone.

17. Composition according to any one of Claims 1 to 16, **characterized in that** the metal oxide nanopigment(s) is(are) present in concentrations ranging from 0.1 to 30% by weight, and preferably from 0.5 to 10% by weight, relative to the total weight of the composition.

18. Composition according to any one of Claims 1 to 17, **characterized in that** it has a pH ranging from 3.5 to 11, preferably from 5.5 to 11 and even more preferably from 5.5 to 8.5.

19. Composition according to any one of Claims 1 to 18, **characterized in that** it is in the form of an emulsion, a lotion, a gel, a vesicle dispersion, a paste or a powder or is packaged as an aerosol and is in the form of a mousse or a spray.

20. Composition according to Claim 19, **characterized in that** it is in the form of an emulsion and constitutes a cream-gel.

21. Composition according to any one of Claims 1 to 20, **characterized in that** the cosmetically acceptable medium consists of water or of water and at least one organic solvent chosen from the group consisting of hydrophilic, lipophilic and amphiphilic organic solvents, or mixtures thereof.

22. Composition according to any one of Claims 1 to 21, **characterized in that** it also comprises at least one common cosmetic adjuvant chosen from fatty substances, standard gelling and/or thickening agents, anionic polymers other than the acrylic terpolymer defined in Claim 1, surfactants, moisturizers, emollients, sunscreens, common metal oxide pigments, hydrophilic or lipophilic active agents such as ceramides or dihydroxyacetone, anti-free-radical agents, sequestering agents, antioxidants, preserving agents, acidifying or basifying agents, fragrances, fillers, dyestuffs, silicones and reducing agents.

23. Cosmetic, non-therapeutic treatment process for protecting the skin, the hair, the eyelashes, the eye-brows or mucous membranes, **characterized in that** an effective amount of a composition as defined according to any one of Claims 1 to 22 is applied to these materials.
